# EUROPEAN PATENT SPECIFICATION

(11) **EP 3 227 022 B1**
(45) Date of publication and mention of the grant of the patent: **22.07.2020**
(21) Application number: 15804134.3
(22) Date of filing: 03.12.2015
(51) Int. Cl.: B01J 37/02, B01J 37/08, C07C 67/02, B01J 32/00

(54) **PROCESSES FOR PREPARING A CATALYST, AN AROMATIC CARBONATE USING THE SAME AND A POLYCARBONATE**
VERFAHREN ZUR HERSTELLUNG EINES KATALYSATORS, EINES AROMATISCHEN CARBONATS UNTER VERWENDUNG DES GLEICHEN UND EINES POLYCARBONATS
PROCÉDÉS DE PRÉPARATION D'UN CATALYSEUR, D'UN CARBONATE AROMATIQUE L'UTILISANT ET D'UN POLYCARBONATE

(30) Priority: 04.12.2014 EP 14196231
(43) Date of publication of application: 11.10.2017
(73) Proprietor: Shell Internationale Research Maatschappij B.V., 2596 HR The Hague (NL)
(72) Inventor: VAPORCIYAN, Garo Garbis, Houston, Texas 77079 (US); YU, Kunquan, 1031 HW Amsterdam (NL)
(74) Representative: Shell Legal Services IP
(86) International application number: PCT/EP2015/078456
(87) International publication number: WO 2016/087559

(56) References cited:
- US-A- 4 201 721
- US-A- 5 210 268

## Description

### Field of the invention

The present invention relates to a process for preparing a catalyst involving a catalyst carrier drying step; to a process for preparing an aromatic carbonate, such as a diaryl carbonate, using the catalyst thus prepared or dried; and to a process for making a polycarbonate from the diaryl carbonate thus prepared.

### Background of the invention

It is known to produce aromatic carbonates from a dialkyl carbonate and an aryl alcohol. For example, the aromatic carbonate may be a diaryl carbonate, such as diphenyl carbonate, which may be prepared from a dialkyl carbonate and an aryl alcohol. In such processes, the dialkyl carbonate is converted into diaryl carbonate via the following steps. In a first step, transesterification of the dialkyl carbonate with the aryl alcohol takes place to yield alkyl aryl carbonate (also an aromatic carbonate) and alkyl alcohol. In a second step, disproportionation of the alkyl aryl carbonate takes place to yield diaryl carbonate and dialkyl carbonate. Further transesterification of the alkyl aryl carbonate with aryl alcohol yielding diaryl carbonate and alkyl alcohol may also take place.

For example, US5210268 discloses a process for producing an aromatic carbonate or aromatic carbonate mixture by a transesterification reaction between a starting material selected from a dialkyl carbonate, an alkyl aryl carbonate and a mixture thereof and a reactant selected from an aromatic hydroxy compound, an alkyl aryl carbonate and a mixture thereof, wherein the starting material and the reactant are continuously fed to a continuous multistage distillation column to effect a transesterification reaction. Furthermore, a process for making an aromatic polycarbonate is as well disclosed in said document.

WO2011067263 discloses a process for preparing a diaryl carbonate (preferably diphenyl carbonate) from a dialkyl carbonate (such as dimethyl carbonate or diethyl carbonate) and an aryl alcohol (preferably phenol), wherein the catalyst may be one selected from a wide variety of catalysts. For example, said WO2011067263 discloses the use of a compound of formula TiX₄, wherein X may be an acetoxy, alkoxy, arylalkoxy or aryloxy group. Said compound of formula TiX₄ may be used as a homogeneous catalyst. However, before use as a catalyst, a solution comprising said compound of formula TiX₄ may also be used to impregnate a carrier with, resulting in a heterogeneous, titanium containing supported catalyst.

An example of the above-mentioned impregnation is disclosed in WO2011014374. In paragraph [00106] of said WO2011014374, it is disclosed that the supports may require removal of condensed water in the pores prior to contacting organometallic compounds with the supports to perform immobilization, wherein condensed water on a support is defined as water content that may be removed by drying the support at a temperature in the range from about 50 °C to about 400 °C in dry gas flow or under a vacuum, depending upon chemical composition of the support. In Experiment 3 of said WO2011014374, a titanium containing supported catalyst was prepared in the following way (as summarized) involving drying and impregnation as mentioned above. First of all, a granular silica gel (the carrier) was treated with an aqueous sodium hydroxide solution. Such a treatment is intended to increase the number of silanol (Si-OH) groups on the surface of the silica carrier which is beneficial for loading a relatively high amount of titanium on the carrier during the below-mentioned treatment with a titanium n-butoxide solution. Then the silica gel was washed, first with cold water and then with hot water (about 80 °C), to remove trace amounts of sodium on the silica. The resulting treated silica gel was dried at 125 °C for 2 hours and then at 300 °C for 2 hours under nitrogen purge. Further, a titanium n-butoxide solution was prepared by dissolving titanium n-butoxide in dried toluene. This solution was circulated up-flow through a reactor wherein the dried granular silica gel support was loaded. After circulating the titanium n-butoxide solution through the reactor at ambient temperature for 15 minutes, the reactor was heated to 168 °C and the circulation was continued at that temperature for 4.5 hours. After cooling the reactor and draining excess solution from the reactor, the supported catalyst was washed with dry toluene up-flow for 1.5 hours. Finally, the washed catalyst was dried at 168 °C in nitrogen gas (up-flow) for 2 hours.

In practice, catalyst carriers, such as for example the above-mentioned silica, may contain water to some extent, as also recognized in above-discussed WO2011014374. Depending on the moisture content in the atmosphere in which a catalyst carrier is stored, the water content may be up to 15 wt.%, typically 1-3 wt.%. Further, water may originate from any carrier pre-treatment process including the above-mentioned treatment with an aqueous sodium hydroxide solution followed by one or more water wash steps. The presence of water in the carrier may be problematic in that this water may react with a metal containing compound, such as above-mentioned TiX₄, used to impregnate the carrier with and/or may lead to undesired reactions in any subsequent reaction step wherein the catalyst is used, such as in the above-mentioned process for preparing an aromatic carbonate from a dialkyl carbonate and an aryl alcohol.

For example, in a case where said TiX₄ is titanium tetraalkoxide (Ti(OR)₄), water reacts with said compound in the following way:

Ti(OR)₄ + 4H₂O → Ti(OH)₄ + 4ROH (hydrolysis)

Ti(OH)₄ → TiO_{2˙}xH₂O + (2-x)H₂O (condensation)

Thus, said reaction results in the production of titanium dioxide of formula TiO₂, also known as titanium(IV) oxide or titania. The presence of such TiO₂ is disadvantageous as it has little to no catalytic activity, more especially in the above-mentioned process for preparing aromatic carbonates from a dialkyl carbonate and an aryl alcohol. Therefore, it is desirable to prevent the formation of TiO₂, and therefore the loss of valuable Ti metal, as much as possible. Furthermore, TiO₂ is a powder that is not soluble in most solvents. Therefore, another disadvantage of TiO₂ formation is that this powder covers the surface of the intended catalyst carrier thereby blocking access to the catalyst carrier pores for the impregnation solution containing the metal containing compound, so that only a relatively small part of the internal and external surface of the catalyst carrier is impregnated with said solution. In summary, the presence of water may result in a catalyst the activity of which may be far less than the desired activity level.

Furthermore, the presence of water may be problematic during use of the catalyst. For example, in a case where a heterogeneous catalyst is used, for example a titanium containing supported catalyst such as the catalyst as prepared in Experiment 3 of above-mentioned WO2011014374, water may be introduced for a variety of reasons. For example, if during maintenance the column containing such catalyst has to be opened, the catalyst may come into contact with moisture from the air and thereby retain a certain amount of water. This water may then react with metal containing groups, such as -(OR)₃Ti-O-Si- groups, in such way that Ti(OH)₄ is formed which may be further converted into TiO₂ powder as illustrated above. In addition, when during use of such supported catalyst containing a certain amount of water, also a homogeneous catalyst is used, such as a Ti(OR)₄ containing solution, the above-mentioned hydrolysis and condensation reactions resulting in TiO₂ powder may also take place.

Therefore, in practice, there is a need to remove water from a carrier before impregnation is performed and the catalyst is prepared. Likewise, there is a need to remove water from a supported catalyst after the catalyst has been prepared. Above-mentioned WO2011014374 teaches to remove such water, before impregnation of the carrier, by drying at 125 °C for 2 hours and then at 300 °C for 2 hours under nitrogen purge. It is a disadvantage that such high temperature is needed to effect the drying. A further disadvantage is that a nitrogen gas stream is to be used. A first disadvantage associated with the use of a nitrogen gas stream is that costly storage for nitrogen gas is required. Secondly, the use of nitrogen gas stream results in a stream comprising nitrogen and water, from which water needs to be separated before the nitrogen could be recycled in which separation a compressor is needed. Alternatively, if there is no recycle of nitrogen but nitrogen is used once-through in the drying process, this would result in loss of valuable nitrogen but also in the need to scrub the used nitrogen stream before the nitrogen gas may be vented into the air. All of the foregoing options result in additional equipment for storing nitrogen gas, for separating water from used nitrogen gas and for scrubbing used nitrogen gas before venting into the air. Thirdly, a further disadvantage associated with the use of a nitrogen gas stream is that nitrogen gas is not a very efficient drying agent as it is a gas which has to remove a liquid (water). Interactions between a gas and a liquid are generally relatively less strong. A still further disadvantage is that it is cumbersome and complicated to measure water content in a nitrogen gas stream that has been used as a drying agent, so as to determine whether the drying is completed. Easy water content determination methods, like Karl Fischer methods, cannot be applied to nitrogen gas streams.

Furthermore, in the above-described catalyst preparation process disclosed in WO2011014374, the dried carrier is impregnated with a titanium n-butoxide solution in toluene. Finally, said carrier is dried at 168 °C in nitrogen gas (up-flow) for 2 hours. Such drying (toluene removal) step is disadvantageous in that a high temperature needs to be applied and nitrogen gas is to be used. Reference is also made to the above discussion of disadvantages associated with the use of nitrogen gas as a drying agent. Furthermore, the use of toluene is generally undesired as it is flammable and therefore a hazardous chemical. It is not desired to have an additional hydrocarbon inventory (storage) on a chemical production site, especially if the hydrocarbon in question is hazardous, like toluene. Further, the nitrogen gas stream resulting from such toluene removal step will have to be sent to a scrubber system before venting the nitrogen gas into the air and the remaining toluene will have to be burnt. This would result in the loss of both nitrogen gas (valuable drying agent) and toluene (valuable solvent). Toluene should be removed since the presence thereof in any subsequent step wherein the catalyst is used to catalyze a certain reaction, should be minimized, such as in the above-mentioned process for preparing an aromatic carbonate from a dialkyl carbonate and an aryl alcohol. It is difficult to completely remove the toluene out of the system once the main desired reaction (e.g. making an aromatic carbonate) starts and this poses a risk in that the toluene would contaminate various process streams resulting from such main reaction. More in particular, in the preparation of an aromatic carbonate from a dialkyl carbonate and an aryl alcohol, said dialkyl carbonate (one of the starting materials) is generally used as the solvent and it is desired that no other solvent is introduced which does not correspond to any one of the starting materials in or products from such diaryl carbonate production process. Therefore, it is also desired to omit the use of a solvent like toluene or any other solvent in the catalyst preparation process which solvent would have to be completely removed before use of the catalyst.

It is an object of the present invention to provide a process involving drying a catalyst carrier and preparing a catalyst on which a metal is supported, and a process for preparing a catalyst from such dried catalyst carrier, which catalyst may be used in a process for preparing aromatic carbonates from a dialkyl carbonate and an aryl alcohol, wherein the catalyst comprises a carrier on which a metal is supported and wherein the catalyst is prepared by impregnating the dried carrier with a solution wherein a compound containing the metal is dissolved, in which process one or more of the above-mentioned disadvantages do not occur or occur to a lesser extent.

### Summary of the invention

Surprisingly it was found that such catalyst preparation process can be achieved by contacting the carrier with a drying agent which comprises an organic carbonate.

The present invention relates to a process for preparing a catalyst which comprises a carrier on which a metal is supported, said process comprising drying the carrier by contacting the carrier with a drying agent which comprises an organic carbonate resulting in a dried carrier; and impregnating the dried carrier with a solution wherein a compound containing the metal is dissolved in a solvent which is an organic carbonate or an alcohol.

The drying process of the present invention surprisingly does not have many of the above-mentioned drawbacks as identified above in relation to the prior art drying process. Further, it is an additional advantage of the present invention that both the drying agent used in the above-mentioned drying process and drying step of the catalyst preparation process, and the solvent used in the impregnation step of the catalyst preparation process may be an organic carbonate, preferably the same organic carbonate, and may therefore be chosen to be the same as the dialkyl carbonate to be used in any subsequent step of preparing an aromatic carbonate by reacting a dialkyl carbonate and an aryl alcohol in the presence of the catalyst thus prepared or dried.

For it has surprisingly appeared that an organic carbonate, such as a dialkyl carbonate, is a good catalyst carrier drying agent, as good as or better than conventional drying agents such as a nitrogen gas stream. Organic carbonates have a relatively high water absorption capacity and remove water relatively fast. Furthermore, it has surprisingly appeared that such organic carbonate is also a good solvent for preparing a solution wherein the metal containing compound is dissolved, with which solution the dried catalyst carrier is to be impregnated, as good as or better than conventional solvents such as toluene. Since in the catalyst preparation process the organic carbonate (drying agent and solvent) may be the same as the dialkyl carbonate to be used in any subsequent step of preparing an aromatic carbonate by reacting a dialkyl carbonate and an aryl alcohol in the presence of the catalyst thus prepared, organic carbonate used in the impregnation step need not be removed. This leads to a simplified, time-saving and more efficient catalyst preparation and start-up procedure. In addition, the present invention advantageously avoids the use of solvents other than organic carbonates, which other solvents may be flammable, such as toluene.

Alternatively, in the present invention, the solvent for the impregnation solution may be an alcohol which still results in many of the above-mentioned advantages discussed in connection with the use of an organic carbonate in the drying step. Besides, such alcohol may advantageously be chosen to be the same as an alcohol to be used or as formed in any subsequent step of preparing an aromatic carbonate by reacting a dialkyl carbonate and an aryl alcohol in the presence of the catalyst thus prepared. For example, an aryl alcohol (like phenol) may be used as such impregnation solvent. Alternatively, an alkyl alcohol may be used as such impregnation solvent, preferably an alkyl alcohol which is the same as the alkyl alcohol that is produced when preparing an aromatic carbonate in aforementioned way, for example ethanol in case diethyl carbonate is used as one of the starting materials.

Further, the present invention relates to a process for preparing an aromatic carbonate, which may be an alkyl aryl carbonate or a diaryl carbonate, using the catalyst prepared in accordance with the catalyst preparation process of the present invention or the catalyst dried in accordance with the drying process of the present invention. Still further, the present invention relates to a process for making a polycarbonate from a diaryl carbonate prepared in accordance with the aromatic carbonate preparation process of the present invention.

### Brief description of the drawing

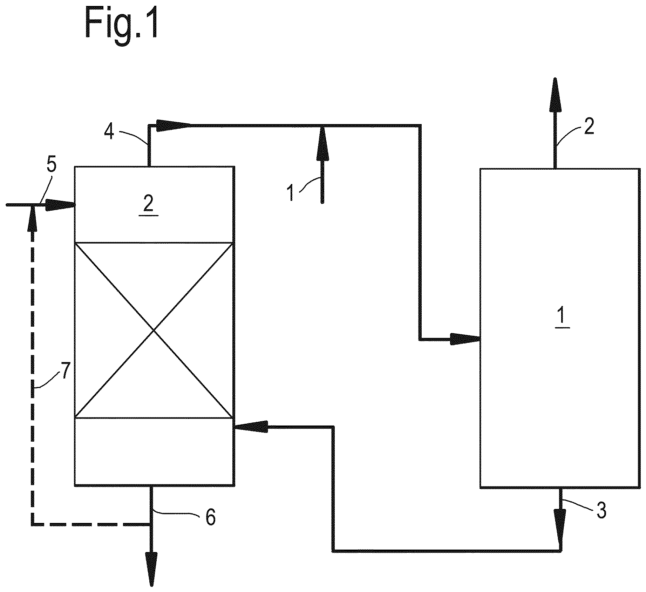

Figure 1 shows an embodiment of the present invention.

### Detailed description of the invention

In the catalyst preparation process of the present invention, the carrier is contacted with a drying agent which comprises an organic carbonate.

Said catalyst carrier may be any carrier. Preferably, said carrier is a porous, inorganic carrier. Further, preferably, the surface of the carrier contains hydroxyl groups, alkoxy groups or a mixture of these groups, more preferably hydroxyl groups. Suitable carriers which may contain hydroxyl groups and/or alkoxy groups at their surface are metal oxide carriers, zeolitic materials and carbonaceous materials. Suitable examples of zeolitic materials are MCM-41, MCM-48 and SBA-15. If the carrier is aforementioned metal oxide carrier, the metal oxide is preferably a metal oxide selected from the group consisting of silica, alumina, zirconia, titania, vanadium oxide and molybdenum oxide, more preferably silica, alumina and zirconia, even more preferably silica and alumina, or mixtures thereof, such as silica-alumina. Most preferably, the carrier is silica. A particular example of silica is silica gel.

The catalyst carrier may have any form. It may be in the form of pellets, extrudates, spheres, granules, honeycomb, and the like, in sizes ranging from 1 mm to 5 mm for various fixed bed reactors. Alternatively one may choose to use woven cloth or mesh made out of fiberglass or carbon fiber or both as support along with structured packing materials, which are suitably shaped and sized properly depending on type of reactors. Supports in powder or microsphere forms may also be used for the preparation of catalysts to be used for slurry or stirred reactor.

Further properties of a porous catalyst carrier which may be used in the present invention, such as BET surface area ("BET" = Brunauer-Emmett-Teller), pore volume and average pore diameter, may vary within wide ranges. For example, the BET surface area may be of from 50 to 700 m²/g, the pore volume may be of from 0.4 to 1.0 cm³/g and/or the average pore diameter may be of from 50 to 500 Å. There is a wide variety of commercially available carriers which have one or more of the aforementioned properties.

Further, in the present invention, the drying agent used to dry the above-described catalyst carrier comprises an organic carbonate. Any organic carbonate may be used. For drying to take place, generally, the water content of the organic carbonate before drying should be lower than the final water content that one wishes to achieve for the catalyst carrier or catalyst to be dried. Thus, if some water is present in the organic carbonate before drying, the water content of that organic carbonate, being dependent on the target water content for the catalyst carrier, may vary within broad ranges. Suitably, before drying, the organic carbonate does not contain water or it has a water content of at most 1 wt.% (= 10,000 parts per million by weight (ppmw)), more suitably at most 5,000 ppmw, more suitably at most 2,000 ppmw, more suitably at most 1,000 ppmw, more suitably at most 500 ppmw, more suitably at most 200 ppmw, more suitably at most 100 ppmw, more suitably at most 50 ppmw, more suitably at most 20 ppmw, most suitably at most 10 ppmw.

Preferably, the organic carbonate drying agent contains no or substantially no catalyst. Such catalyst may for example be the catalyst as further defined hereinbelow, that is to say a metal containing compound which, in addition to the metal, contains one or more ligands, which may be the same or different and one or more of which ligands are preferably selected from the group consisting of alkoxy, arylalkoxy, aryloxy, alkylaryloxy, alkyl, arylalkyl, aryl, alkylaryl, hydroxide, carboxylate, carbonate and halide groups. Within the present specification, by "substantially no" in relation to the amount of a specific component, such as said catalyst, it is meant an amount which is at most 1,000, preferably at most 500, more preferably at most 100, more preferably at most 50, more preferably at most 30, more preferably at most 20, and most preferably at most 10 ppmw (parts per million by weight) of the component in question, based on the total amount (i.e. weight).

Further, the catalyst carrier may have any water content, for example up to 15 wt.%, typically 1-3 wt.%. The target water content for the catalyst carrier that may be achieved depends on the water content of the organic carbonate drying agent, as explained above. For example, by carrying out the drying process or the drying step of the catalyst preparation process of the present invention, a dried catalyst carrier or dried catalyst may be obtained having a water content of at most 1 wt.% (= 10,000 parts per million by weight (ppmw)), more suitably at most 5,000 ppmw, more suitably at most 2,000 ppmw, more suitably at most 1,000 ppmw, more suitably at most 500 ppmw, more suitably at most 200 ppmw, more suitably at most 100 ppmw, more suitably at most 50 ppmw, more suitably at most 20 ppmw, most suitably at most 10 ppmw.

As to the chemical composition of the organic carbonate drying agent, any organic carbonate may be used. For example, the organic carbonate may be a compound of formula ROC(=O)OR', wherein R and R' may be the same or different and are each an alkyl or aryl group, that is to say a compound selected from the group consisting of dialkyl carbonates, diaryl carbonates and alkyl aryl carbonates. Said alkyl group may have 1 to 4, suitably 1 to 3 carbon atoms. Suitably, said alkyl group is a methyl group or ethyl group, more suitably an ethyl group. Said aryl group may have 6 to 12 carbon atoms. Suitably, said aryl group is a phenyl group. A suitable example of said diaryl carbonate is diphenyl carbonate. Suitable examples of said alkyl aryl carbonate are methyl phenyl carbonate and ethyl phenyl carbonate. Preferably, said organic carbonate of formula ROC(=O)OR' is a dialkyl carbonate wherein R and R' are C₁₋₄ alkyl groups, preferably C₁₋₃ alkyl groups. More preferably, said dialkyl carbonate is dimethyl carbonate or diethyl carbonate, most preferably diethyl carbonate. Further, the organic carbonate may be a cyclic carbonate, like an alkylene carbonate, for example an alkylene carbonate having 3 to 6, suitably 3 to 4 carbon atoms. Suitable examples of alkylene carbonates are ethylene carbonate and propylene carbonate

In the catalyst preparation process of the present invention, a catalyst is prepared which comprises a carrier on which a metal is supported. After the drying step of that process, the dried carrier is impregnated with a solution wherein a compound containing the metal is dissolved in a solvent which is an organic carbonate or an alcohol.

Preferably, the catalyst to be prepared is a catalyst suitable for use in a process for preparing an aromatic carbonate, comprising reacting a dialkyl carbonate or an alkyl aryl carbonate with an aryl alcohol or an alkyl aryl carbonate, resulting in an aromatic carbonate which is an alkyl aryl carbonate or a diaryl carbonate. There is a wide variety of catalysts which can be used in such aromatic carbonate production process. For example, reference is made to the passage at page 4, line 31 to page 6, line 29 of above-mentioned WO2011067263. In the catalyst preparation process of the present invention, it is especially preferred that the metal containing compound dissolved in the solution with which the dried catalyst carrier is impregnated, is a metal containing compound which is sensitive to the presence of water in the catalyst carrier. Such sensitivity may manifest itself by reaction of the metal and/or any ligand with water. For example, ligands, such as for example an alkoxide ligand, may react with water.

Consequently, in the present invention, said metal containing compound is a compound which, in addition to the metal, contains one or more ligands, which may be the same or different and one or more of which ligands are preferably selected from the group consisting of alkoxy, arylalkoxy, aryloxy, alkylaryloxy, alkyl, arylalkyl, aryl, alkylaryl, hydroxide, carboxylate, carbonate and halide groups. An "alkoxy" group is a group of formula R-O⁻ wherein R is an alkyl group. An "arylalkoxy" group is a group of formula Ar-R-O⁻ wherein Ar is an aryl group and R is an alkyl group. An "aryloxy" group is a group of formula Ar-O⁻ wherein Ar is an aryl group. An "alkylaryloxy" group is a group of formula R-Ar-O⁻ wherein R is an alkyl group and Ar is an aryl group. An "alkyl" group is of formula R. An "arylalkyl" group is a group of formula Ar-R wherein Ar is an aryl group and R is an alkyl group. An "aryl" group is a group of formula Ar. An "alkylaryl" group is a group of formula R-Ar wherein R is an alkyl group and Ar is an aryl group. A "hydroxide" group is a group of formula HO⁻. A carboxylate group is a group of formula R'-C(=O)-O⁻ wherein R' may be an alkyl, arylalkyl, aryl or alkylaryl group. For example, said carboxylate group may be an acetoxy group. A carbonate group is a group of formula ⁻O-C(=O)-O⁻. The nature of the alkyl and aryl groups which make up said alkoxy, arylalkoxy, aryloxy, alkylaryloxy, alkyl, arylalkyl, aryl, alkylaryl and carboxylate groups is not essential. These alkyl and aryl groups may be substituted or unsubstituted. Further, the alkyl group may be a branched or linear, preferably linear, C₁-C₆ alkyl group, preferably C₁-C₄ alkyl group, more preferably C₁-C₂ alkyl group (methyl or ethyl group), most preferably C₂ alkyl group (ethyl group). The aryl group may be a phenyl group. A halide group may be selected from the group consisting of fluoride (F⁻), chloride (Cl⁻) , bromide (Br⁻) and iodide (I⁻).

Preferably, one or more of said ligands are selected from the group consisting of alkoxy, arylalkoxy, aryloxy, alkylaryloxy, alkyl, arylalkyl, aryl, alkylaryl, hydroxide, carboxylate, carbonate and halide groups, more preferably from the group consisting of alkoxy, arylalkoxy, aryloxy, alkylaryloxy, alkyl, arylalkyl, aryl, alkylaryl and hydroxide groups, more preferably from the group consisting of alkoxy, arylalkoxy, aryloxy and alkylaryloxy groups, more preferably from the group consisting of alkoxy and arylalkoxy groups, most preferably from the group consisting of alkoxy groups. Thus, if an alkoxy group is selected, it may be a branched or linear, preferably linear, C₁-C₆ alkoxy group, preferably C₁-C₄ alkoxy group, more preferably C₁-C₂ alkoxy group (methoxy or ethoxy group), most preferably C₂ alkoxy group (ethoxy group).

The metal containing compound may contain one or more different metals. Said metal(s) may be in an oxidized state, in which case the same metal may have one oxidation state or two or more different oxidation states. For example, in the case where the metal containing compound contains one metal having one oxidation state and one or more negatively charged ligands, said compound may be of the following formula:

M^{m+}(Lₙ)^{l-}

wherein:
M is the metal;
m is an integer which may be 1, 2, 3 or 4, suitably 2, 3 or 4, more suitably 3 or 4, most suitably 4;
L is the ligand;
1 is an integer which may be 1, 2, 3 or 4, suitably 1, 2 or 3, more suitably 1 or 2, most suitably 1; and
m is the product of n and 1 (m=n^{∗}l) so that the compound is electrically neutral.

The metal may be any metal which in an oxidized state can form a compound which, in addition to the metal, contains one or more ligands, which may be the same or different and one or more of which ligands are preferably selected from the group consisting of alkoxy, arylalkoxy, aryloxy, alkylaryloxy, alkyl, arylalkyl, aryl, alkylaryl, hydroxide, carboxylate, carbonate and halide groups, wherein the ligands are as defined above. For example, the metal may be a metal selected from the group consisting of groups 2, 3, 4, 5, 6, 12, 13, 14, 15 and 16 of the periodic table of the chemical elements, suitably groups 4 and 14 thereof. Examples of suitable active metals from these groups include magnesium (Mg), calcium (Ca), lanthanum (La), actinium (Ac), titanium (Ti), zirconium (Zr), hafnium (Hf), vanadium (V), niobium (Nb), tantalum (Ta), chromium (Cr), molybdenum (Mo), tungsten (W), zinc (Zn), tin (Sn), lead (Pb) and antimony (Sb). Preferably, said metal is selected from the group consisting of titanium (Ti), lead (Pb) and tin (Sn). More preferably, the metal is titanium. Said titanium may be used in oxidation state +3 or +4, suitably +4. An example of such titanium containing compound is titanium tetraalkoxide (Ti(OR)₄), wherein the alkoxide is as defined above, such as titanium tetraethoxide (Ti(OEt)₄). Other suitable examples of titanium containing compounds are titanium tetramethoxide, titanium tetrapropoxide, titanium tetrabutoxide and titanium tetraphenoxide. Suitable examples of tin containing compounds include tin alkoxides, alkyl tin alkoxides, alkyl tin oxides and alkyl tin hydroxides.

In the present catalyst preparation process, the metal containing compound used in the impregnation step is dissolved in a solvent which is an organic carbonate or an alcohol. Preferably, said solvent is an organic carbonate. In case the solvent used in the impregnation step is an alcohol, any alcohol may be used. Preferably, said alcohol is an alkyl alcohol or an aryl alcohol, more preferably an alkyl alcohol. An "alkyl alcohol" is a compound of formula R-OH wherein R is an alkyl group. An "aryl alcohol" is a compound of formula Ar-OH wherein R is an aryl group. The nature of the alkyl and aryl groups which make up said alkyl alcohol and aryl alcohol is not essential. These alkyl and aryl groups may be substituted or unsubstituted. Further, the alkyl group may be a branched or linear, preferably linear, C₁-C₆ alkyl group, preferably C₁-C₄ alkyl group, more preferably C₁-C₂ alkyl group (methyl or ethyl group), most preferably C₂ alkyl group (ethyl group). The aryl group may be a phenyl group.

In case the solvent used in the impregnation step is an organic carbonate, any organic carbonate may be used. The above description of the organic carbonate used as drying agent in the drying step of the present catalyst preparation process also applies to the organic carbonate used as solvent in the impregnation step of the same process. Preferably, the organic carbonate used in the drying step and the organic carbonate used in the impregnation step are the same.

Suitably, the organic carbonate or alcohol solvent used in the impregnation step does not contain water or it has a water content of at most 1 wt.% (= 10,000 parts per million by weight (ppmw)), more suitably at most 5,000 ppmw, more suitably at most 2,000 ppmw, more suitably at most 1,000 ppmw, more suitably at most 500 ppmw, more suitably at most 200 ppmw, more suitably at most 100 ppmw, more suitably at most 50 ppmw, more suitably at most 20 ppmw, most suitably at most 10 ppmw.

The concentration of the metal containing compound in the solution used in the impregnation step is not essential and may vary within wide ranges. For example, said concentration may be of from 500 parts per million by weight (ppmw) to 5 wt.%, suitably 1,000 ppmv to 3 wt.%, based on the metal.

The temperature and pressure in the above-mentioned drying process and drying step and in the impregnation step are not essential and may vary within wide ranges. For example, the temperature in the drying process and drying step may be of from 20 to 300 °C, suitably 100 to 250 °C, more suitably 120 to 200 °C. The pressure in the drying process and drying step may be of from vacuum pressure to 10 bara, suitably 1 to 8 bara, more suitably 1 to 4 bara. Further, for example, the temperature in the impregnation step may be of from 20 to 300 °C, suitably 100 to 250 °C, more suitably 120 to 200 °C. The pressure in the impregnation step may be of from vacuum pressure to 10 bara, suitably 1 to 8 bara, more suitably 1 to 4 bara. It is especially advantageous, that in accordance with the invention, the drying may simply be carried out at ambient temperature and pressure. A further advantage is that in the present invention, the drying and the impregnation may be carried out at the same temperature and pressure, which is therefore preferred.

While any process embodying the present invention is described in terms of "comprising", "containing" or "including" various steps, they can also "consist essentially of" or "consist of" the various described steps.

For example, between the above-mentioned drying and impregnation steps of the catalyst preparation process of the present invention, there need not be any intermediate step. However, if the drying step is for example carried out batchwise, rather than continuously, a possible intermediate step may be the separation of the organic carbonate drying having an increased water content from the dried catalyst carrier. That is to say, in the present invention, each of the drying and impregnation steps may be carried out batchwise or continuously.

An embodiment of a process in accordance with the present invention is illustrated in Figure 1. In the process shown in Figure 1, fresh organic carbonate is fed into distillation column 1 via line 1. Any water in the organic carbonate is removed as overhead via line 2. Dry organic carbonate coming from the bottom of distillation column 1 is fed to the bottom of reactive distillation column 2 via line 3 and flows upwardly through a catalyst bed comprising a wet catalyst carrier. The feed of fresh organic carbonate stops when there is enough liquid levels in both columns 1 and 2. An embodiment wherein dry organic carbonate flows downwardly (not shown in Figure 1), instead of upwardly, through reactive distillation column 2 is also envisaged. Water is removed from the catalyst bed by the dry organic carbonate. Wet organic carbonate coming from the top of reactive distillation column 2 is recycled back to distillation column 1 via line 4 for water removal. Once the catalyst bed is dry, the feed of dry organic carbonate via line 3 is stopped and a solution wherein a metal containing compound is dissolved in an organic carbonate is fed to the top of reactive distillation column 2 via line 5 and flows downwardly through the catalyst bed to impregnate the catalyst carrier. Said solution leaves column 2 via line 6 (once-through operation). Alternatively, said solution in line 6 may be partially or completely sent, via line 7, to line 5 and mixed with fresh feed (recycle operation).

Further, the present invention relates to a process for preparing an aromatic carbonate, using the catalyst prepared in accordance with the catalyst preparation process of the present invention or the catalyst dried in accordance with the drying process of the present invention. Accordingly, the present invention relates to a process for preparing an aromatic carbonate, comprising reacting a dialkyl carbonate or an alkyl aryl carbonate with an aryl alcohol or an alkyl aryl carbonate, in the presence of a catalyst prepared in accordance with the above-described catalyst preparation process or a catalyst dried in accordance with the above-described drying process, resulting in an aromatic carbonate which is an alkyl aryl carbonate or a diaryl carbonate. Further, accordingly, the present invention relates to a process for preparing an aromatic carbonate, comprising preparing a catalyst in accordance with the above-described catalyst preparation process or drying a catalyst in accordance with the above-described drying process, and reacting a dialkyl carbonate or an alkyl aryl carbonate with an aryl alcohol or an alkyl aryl carbonate, in the presence of the catalyst thus prepared or dried, resulting in an aromatic carbonate which is an alkyl aryl carbonate or a diaryl carbonate. The embodiments and preferences as described above with reference to the catalyst preparation and drying processes of the present invention also apply to said catalyst preparation or drying step of the aromatic carbonate preparation process of the present invention.

In the aromatic carbonate preparation process of the present invention, the alkyl group in the dialkyl carbonate and alkyl aryl carbonate may have 1 to 4, suitably 1 to 3 carbon atoms. Suitably, said alkyl group is a methyl group or ethyl group, more suitably an ethyl group. Further, in the aromatic carbonate preparation process of the present invention, the aryl group in the aryl alcohol, alkyl aryl carbonate and diaryl carbonate may have 6 to 12 carbon atoms. Preferably, said aryl group is a phenyl group. Therefore, preferably, said aryl alcohol is phenol and said diaryl carbonate is diphenyl carbonate. Suitable examples of said alkyl aryl carbonate are methyl phenyl carbonate and ethyl phenyl carbonate. Preferably, said dialkyl carbonate is of formula ROC(=O)OR', wherein R and R' may be the same or different and are C₁₋₄ alkyl groups, preferably C₁₋₃ alkyl groups. More preferably, said dialkyl carbonate is dimethyl carbonate or diethyl carbonate, most preferably diethyl carbonate. Further, preferably, in the aromatic carbonate preparation process of the present invention, a dialkyl carbonate is reacted with an aryl alcohol resulting in the corresponding alkyl aryl carbonate.

In the aromatic carbonate preparation process of the present invention, the catalyst is a catalyst prepared in accordance with the above-described catalyst preparation process wherein the solvent used in the impregnation step of the catalyst preparation process is an organic carbonate. It is further preferred that the organic carbonate used in the drying step of the catalyst preparation process and the organic carbonate used in the impregnation step of the catalyst preparation process are the same, and that said organic carbonate is a dialkyl carbonate, an alkyl aryl carbonate or a diaryl carbonate, more preferably a dialkyl carbonate.

Advantageously, in the aromatic carbonate preparation process of the present invention, the same process configuration can be applied as the process configuration applied in the preceding catalyst preparation or drying process of the present invention. For example, in a case where a dialkyl carbonate is reacted with an aryl alcohol resulting in the corresponding alkyl aryl carbonate, and the dialkyl carbonate used in the aromatic carbonate preparation process and the organic carbonate used in the drying process or in the drying and impregnation steps of the catalyst preparation process are the same, the same process configuration as shown in Figure 1 can be applied to the aromatic carbonate preparation process of the present invention, with the proviso that in such case a bottom stream comprising alkyl aryl carbonate, optionally diaryl carbonate, and phenol leaves reactive distillation column 1 via line 6, the top stream from said column in line 4 comprises dialkyl carbonate and alkyl alcohol rather than dialkyl carbonate and water, and accordingly the top stream leaving distillation column 1 via line 2 comprises alkyl alcohol rather than water. Further, via line 1, fresh dialkyl carbonate is fed. Optionally, via line 5, a homogeneous catalyst solution may be fed. Recycle line 7 would not be used in the aromatic carbonate preparation process.

To complete the conversion of a dialkyl carbonate and an aryl alcohol into a diaryl carbonate through the intermediate formation of an alkyl aryl carbonate, a series of two or three, preferably three, reactive distillation columns in total may be applied. The various embodiments as disclosed in above-mentioned WO2011067263, disclosing a process wherein three reactive distillation columns are used, may be applied to the present aromatic carbonate preparation process. The disclosure of WO2011067263 is herein incorporated by reference.

The pressures in said three reactive distillation columns may vary within wide limits. The pressure at the top of the first reactive distillation column may be 2 to 7 bar, preferably 2.5 to 5 bar. The pressure at the top of the second reactive distillation column may be 0.1 to 3 bar, preferably 0.3 to 1.5 bar. The pressure at the top of the third reactive distillation column may be 10 to 600 mbar, preferably 20 to 500 mbar. Preferably, the pressure at the top of the first reactive distillation column is higher than that of the second reactive distillation column which in turn is higher than that of the third reactive distillation column.

The temperatures in said three reactive distillation columns may also vary within wide limits. The temperature at the bottom of the first, second and third reactive distillation columns may be 50 to 350 °C, preferably 120 to 280 °C, more preferably 150 to 250 °C, most preferably 160 to 240 °C.

The catalyst in one or more of said three reactive distillation columns may be a catalyst prepared in accordance with the catalyst preparation process of the present invention. These catalysts are heterogeneous catalysts. In addition, a homogeneous catalyst may be used, with the proviso that at least 1 of these reactive distillation columns, preferably the first one, also contains said heterogeneous catalyst. Such homogeneous catalyst may be added by feeding a solution wherein a compound containing a metal is dissolved in a solvent which is an organic carbonate or an alcohol, as described above with reference to the catalyst preparation process of the present invention.

Still further, the present invention relates to a process for making a polycarbonate from a diaryl carbonate prepared in accordance with the aromatic carbonate preparation process of the present invention. Accordingly, the present invention relates to a process for making a polycarbonate, comprising reacting a dihydroxy aromatic compound with a diaryl carbonate prepared in accordance with the above-described aromatic carbonate preparation process. Further, accordingly, the present invention relates to a process for making a polycarbonate, comprising preparing a diaryl carbonate in accordance with the above-described aromatic carbonate preparation process, and reacting a dihydroxy aromatic compound with the diaryl carbonate thus obtained. The embodiments and preferences as described above with reference to the aromatic carbonate preparation process of the present invention also apply to said diaryl carbonate preparation step of the polycarbonate make process of the present invention.

Further, preferably, said dihydroxy aromatic compound is bisphenol A, which is 4,4'-(propan-2-ylidene)diphenol. The production of polycarbonate by the polymerisation of diaryl carbonate with an aromatic dihydroxy compound, such as bisphenol A, is well known. See for example US5747609, WO2005026235 and WO2009010486, the disclosures of which are herein incorporated by reference.

The present invention is further illustrated by the following Examples.

### Examples

In the Example exemplifying the present invention, a reactive distillation column having a diameter of 1 inch was filled with wet silica, containing about 2 wt.% of water (moisture), as catalyst carrier. The silica used was a silica having a BET surface area of 400 m²/g, a pore volume of 0.6 cm³/g and an average pore diameter of 60 Å. Said column was refluxed with dry diethyl carbonate (DEC) fed to the bottom of the column (0.23 kg/hr of DEC feed), said dry DEC having a water content of less than 10 ppmw, to remove the water from the silica bed at a pressure of 2.8 barg and a temperature of 188 °C. Wet DEC was removed from said column as overhead (0.23 kg/hr of overhead flow; 0.45 kg/hr of reflux). This process was carried out till the overhead contained less than 10 ppmw of water. The moisture content in the DEC overhead was measured using a Karl Fischer method which is an easy method of measuring moisture content. After this was achieved, said dry DEC feed was stopped and a solution of titanium tetraethoxide dissolved in dry DEC (1540 ppmw Ti; less than 10 ppmw of water) was then fed into the top of the silica bed at a downward flow rate of 0.14 kg/hr for 6 hours, and at the same temperature and pressure as applied in the drying step (i.e. 188 °C and 2.8 barg, respectively). Then phenol was fed into the column to start the transesterification reaction between phenol and DEC, at a reduced titanium catalyst feed of 0.12 kg/hr.

In the Example which was not in accordance with the present invention (Comparative Example), the same column and silica as described above were used. However, in this case, the wet silica bed was dried with an upwardly flowing nitrogen gas stream for 14 hours at a temperature of 152 °C, a pressure of 1 bara and a flow of 16 Nm³/hr. The nitrogen flow was then stopped to allow the column to cool down to about 50 °C. Then a solution of titanium tetraethoxide dissolved in dry toluene (1.29 wt.% Ti; less than 10 ppmw of water) was fed into the bottom of the silica bed at an upward flow rate of 3.6 kg/hr at a temperature of 50 °C and a pressure of 2.4 barg for 45 minutes, then at a flow rate of 7.3 kg/hr at a temperature of 135 °C and a pressure of 2.4 barg for 6 hours. The solution coming out of the top of the silica bed was recycled to the bottom of the bed (about 4 circulation cycles). Thereafter, the heating of the column was stopped while the circulation of the titanium catalyst solution continued. Once the column was cooled to about 35 °C, the feed of titanium catalyst solution was stopped, and the liquid was drained from the column. Excess titanium was flushed out of the silica bed with toluene at an upflow rate of 1.4 kg/hr for 2 hours. After the toluene was drained from the column, the column was further purged with DEC at an upflow rate of 1.4 kg/hr for 1 hour. The silica catalyst bed was then ready for normal operation for the transesterification reaction between DEC and phenol, such as the operation as described above for the Example exemplifying the present invention.

## Claims

1. Process for preparing a catalyst which comprises a carrier on which a metal is supported, said process comprising drying the carrier by contacting the carrier with a drying agent which comprises an organic carbonate resulting in a dried carrier; and impregnating the dried carrier with a solution wherein a compound containing the metal is dissolved in a solvent which is an organic carbonate or an alcohol.

2. Process according to claim 1, wherein the organic carbonate used as a drying agent is a compound of formula ROC(=O)OR', wherein R and R' may be the same or different and are each an alkyl or aryl group, preferably a dialkyl carbonate wherein R and R' are C₁₋₄ alkyl groups, more preferably dimethyl carbonate or diethyl carbonate.

3. Process according to claim 1, wherein the solvent used in the impregnation step is an organic carbonate.

4. Process according to claim 3, wherein the organic carbonate is a compound of formula ROC(=O)OR', wherein R and R' may be the same or different and are each an alkyl or aryl group, preferably a dialkyl carbonate wherein R and R' are C₁₋₄ alkyl groups, more preferably dimethyl carbonate or diethyl carbonate.

5. Process according to any one of claims 3-4, wherein the organic carbonate used in the drying step and the organic carbonate used in the impregnation step are the same.

6. Process according to any one of claims 1-5, wherein the compound containing the metal used in the impregnation step is a compound which, in addition to the metal, contains one or more ligands, which may be the same or different and one or more of which ligands are selected from the group consisting of alkoxy, arylalkoxy, aryloxy, alkylaryloxy, alkyl, arylalkyl, aryl, alkylaryl, hydroxide, carboxylate, carbonate and halide groups, more preferably from the group consisting of alkoxy and arylalkoxy groups, most preferably from the group consisting of alkoxy groups.

7. Process for preparing an aromatic carbonate, comprising preparing a catalyst in accordance with the process of any one of claims 1-6, and reacting a dialkyl carbonate or an alkyl aryl carbonate with an aryl alcohol or an alkyl aryl carbonate, in the presence of the catalyst thus prepared, resulting in an aromatic carbonate which is an alkyl aryl carbonate or a diaryl carbonate.

8. Process for making a polycarbonate, comprising preparing a diaryl carbonate in accordance with the process of claim 7, and reacting a dihydroxy aromatic compound with the diaryl carbonate thus obtained.

## Patentansprüche

1. Vorgang zum Herstellen eines Katalysators, der eine Trägersubstanz umfasst, auf dem ein Metall gelagert wird, wobei der Vorgang das Trocknen der Trägersubstanz durch Inberührungbringen der Trägersubstanz mit einem Trocknungsmittel umfasst, das ein organisches Carbonat umfasst, was zu einer getrockneten Trägersubstanz führt; und
Imprägnieren der getrockneten Trägersubstanz mit einer Lösung, wobei eine das Metall enthaltende Verbindung in einem Lösungsmittel gelöst ist, das ein organisches Carbonat oder ein Alkohol ist.

2. Vorgang nach Anspruch 1, wobei das als Trocknungsmittel verwendete organische Carbonat eine Verbindung der Formel ROC(=O)OR' ist, wobei R und R' gleich oder verschieden sein können und jeweils eine Alkyl- oder Arylgruppe sind, vorzugsweise ein Dialkylcarbonat, wobei R und R' C₁₋₄-Alkylgruppen sind, stärker bevorzugt Dimethylcarbonat oder Diethylcarbonat.

3. Vorgang nach Anspruch 1, wobei das in dem Imprägnierungsschritt verwendete Lösungsmittel ein organisches Carbonat ist.

4. Vorgang nach Anspruch 3, wobei das organische Carbonat eine Verbindung der Formel ROC(=O)OR' ist, wobei R und R' gleich oder verschieden sein können und jeweils eine Alkyl- oder Arylgruppe sind, vorzugsweise ein Dialkylcarbonat, wobei R und R' C₁₋₄-Alkylgruppen sind, stärker bevorzugt Dimethylcarbonat oder Diethylcarbonat.

5. Vorgang nach einem der Ansprüche 3-4, wobei das in dem Trocknungsschritt verwendete organische Carbonat und das in dem Imprägnierungsschritt verwendete organische Carbonat gleich sind.

6. Vorgang nach einem der Ansprüche 1-5, wobei die Verbindung, die das in dem Imprägnierungsschritt verwendete Metall enthält, eine Verbindung ist, die zusätzlich zu dem Metall einen oder mehrere Liganden enthält, die gleich oder verschieden sein können und einen oder mehrere von den Liganden aus der Gruppe ausgewählt sind, die aus Alkoxy-, Arylalkoxy-, Aryloxy-, Alkylaryloxy-, Alkyl-, Arylalkyl-, Aryl-, Alkylaryl-, Hydroxid-, Carboxylat-, Carbonat- und Halogenidgruppen besteht, stärker bevorzugt aus der Gruppe, die aus Alkoxy- und Arylalkoxygruppen besteht, am stärksten bevorzugt aus der Gruppe, die aus Alkoxygruppen besteht.

7. Vorgang zum Herstellen eines aromatischen Carbonats, umfassend das Herstellen eines Katalysators nach dem Vorgang nach einem der Ansprüche 1-6 und ein Umsetzen eines Dialkylcarbonats oder eines Alkylarylcarbonats mit einem Arylalkohol oder einem Alkylarylcarbonat, in der Gegenwart des so hergestellten Katalysators, das zu einem aromatischen Carbonat führt, das ein Alkylarylcarbonat oder ein Diarylcarbonat ist.

8. Vorgang zum Erzeugen eines Polycarbonats, umfassend das Herstellen eines Diarylcarbonats nach dem Vorgang nach Anspruch 7 und das Umsetzen einer aromatischen Dihydroxyverbindung mit dem so erhaltenen Diarylcarbonat.

## Revendications

1. Procédé de préparation d'un catalyseur qui comprend un support sur lequel un métal est supporté, ledit procédé comprenant le séchage du support en mettant en contact le support avec un agent de séchage qui comprend un carbonate organique résultant en un support séché ; et l'imprégnation du support séché avec une solution dans laquelle un composé contenant le métal est dissous dans un solvant qui est un carbonate organique ou un alcool.

2. Procédé selon la revendication 1, dans lequel le carbonate organique utilisé comme agent de séchage est un composé de formule ROC(=O)OR', R et R' pouvant être identiques ou différents et étant chacun un groupe alkyle ou aryle, de préférence un carbonate de dialkyle dans lequel R et R' sont des groupes alkyle en C₁ à ₄, plus préférablement le carbonate de diméthyle ou le carbonate de diéthyle.

3. Procédé selon la revendication 1, dans lequel le solvant utilisé dans l'étape d'imprégnation est un carbonate organique.

4. Procédé selon la revendication 3, dans lequel le carbonate organique est un composé de formule ROC(=O)OR', R et R' pouvant être identiques ou différents et étant chacun un groupe alkyle ou aryle, de préférence un carbonate de dialkyle dans lequel R et R' sont des groupes alkyle en C₁ à ₄, plus préférablement le carbonate de diméthyle ou le carbonate de diéthyle.

5. Procédé selon l'une quelconque des revendications 3 à 4, dans lequel le carbonate organique utilisé dans l'étape de séchage et le carbonate organique utilisé dans l'étape d'imprégnation sont identiques.

6. Procédé selon l'une quelconque des revendications 1 à 5, dans lequel le composé contenant le métal utilisé dans l'étape d'imprégnation est un composé qui, en plus du métal, contient un ou plusieurs ligands, qui peuvent être identiques ou différents, un ou plusieurs de ces ligands étant choisis dans le groupe constitué des groupes alcoxy, arylalcoxy, aryloxy, alkylaryloxy, alkyle, arylalkyle, aryle, alkylaryle, hydroxyde, carboxylate, carbonate et halogénure, plus préférablement dans le groupe constitué des groupes alcoxy et arylalcoxy, le plus préférablement parmi le groupe consistant en groupes alcoxy.

7. Procédé de préparation d'un carbonate aromatique, comprenant la préparation d'un catalyseur selon le procédé de l'une quelconque des revendications 1 à 6, et la réaction d'un carbonate de dialkyle ou d'un carbonate d'alkyle et d'aryle avec un alcool arylique ou un carbonate d'alkyle et d'aryle, en présence du catalyseur ainsi préparé, résultant en un carbonate aromatique qui est un carbonate d'alkyle aryle ou un carbonate de diaryle.

8. Procédé de fabrication d'un polycarbonate, comprenant la préparation d'un diarylcarbonate conformément au procédé de la revendication 7, et la réaction d'un composé aromatique dihydroxy avec le carbonate de diaryle ainsi obtenu.
